# EUROPEAN PATENT APPLICATION

(11) **EP 4 835 439 A1**
(43) Date of publication of application: **04.11.2026**
(21) Application number: 25305631.1
(22) Date of filing: 02.05.2025
(51) Int. Cl.: G01N 15/08, G01N 33/24

(54) **A ROCK PROPERTIES MEASUREMENT CELL HAVING AN UPSTREAM POROUS BARRIER ELEMENT, RELATED SYSTEM AND METHOD**

(71) Applicant: TotalEnergies OneTech, 92400 Courbevoie (FR)
(72) Inventor: NONO NGUENDJIO, Frank laurel, 64018 PAU CEDEX (FR); BRUGIDOU, Régis, 64018 PAU CEDEX (FR); CAUBIT, Cyril, 64018 PAU CEDEX (FR)
(74) Representative: Lavoix

(57) **Abstract**

The cell (12) comprises a downstream porous barrier element (18), a downstream diffuser (22), an upstream diffuser (20) and a sleeve (24).

It has an upstream porous barrier element (19) comprising:
+ a first upstream channel (45A) configured to retrieve first fluid (F1) and/or second fluid (F2) from the rock sample (28) or to inject first fluid (F1) and/or second fluid (F2) in the rock sample (28) via an upstream bypass (43C),
+ a second upstream channel (45B), opening facing an upstream face (30) of the upstream porous barrier (19) element and being configured to retrieve or to inject only second fluid (F2) in the rock sample (28).

The upstream diffuser (20), the upstream porous barrier element (19), the downstream porous barrier element (18), the downstream diffuser (22) and the sleeve (24) surrounding them delimit a measurement chamber (34) configured to receive a rock sample (28).

## Description

The present invention concerns a rock properties measurement cell, configured to receive a rock sample to carry out measurements in presence of at least a fluid contained in said rock sample, the measurement cell comprising:
- a downstream porous barrier element, permeable to a first fluid and impervious to a second fluid, the downstream porous barrier element defining an upstream face configured to cooperate with a downstream face of the rock sample, a downstream face opposed to the upstream face, and a downstream bypass connecting the upstream face and the downstream face of the downstream porous barrier element;
- a downstream diffuser, comprising a first downstream opening, delimiting, together with the bypass, a first downstream channel configured to retrieve first fluid and/or second fluid from the rock sample or to inject first fluid and/or second fluid in the rock sample, and a second downstream opening forming a second downstream channel, opening facing the downstream face of the downstream porous barrier element and being configured to retrieve only first fluid from the rock sample or to inject only first fluid in the rock sample,
- an upstream diffuser;
- a sleeve ;

In the oil and gas exploration field, it is critical to define the properties of rocks that are located at an exploration site, since the rock local properties impact the overall behavior of the explored site. Such properties can include, for example, capillary pressure, relative permeability or wettability of the rock.

In order to define properties of the rock, a rock sample is in general extracted from the exploration site and several tests are conducted on the rock sample mounted in a rock properties measurement cell, in order to define the rock properties.

A full cycle of capillary pressure measurements of a reservoir rock is a direct and important input for reservoir engineers to predict fluids distribution in the reservoir and forecast oil production during an assisted oil recovery. These properties depend mostly on wettability, and wettability is a very important parameter that helps understanding how multiphase flow occurs in a petroleum reservoir.

In general, capillary pressure curves are either measured with representative samples of rock in laboratories or numerically derived from a history matching process of relative permeability experiments.

Numerically derived capillary pressure curves are generally not reliable, as there exist many different curves that could history match experimental data from a relative permeability experiment. The best way to proceed is thus to measure the capillary pressure in a lab.

Capillary pressure curves are measured using either a centrifuge technique or a porous plate technique.

The centrifuge technique generally involves rotating the sample in a cell at high speeds for extended periods and recovering the fluid produced by the sample until no production is observed. It thus requires very specific expensive apparatus and complex monitoring. In addition, these devices do not allow pressure and temperature conditioning of the sample during measurements.

The porous plate technique is generally carried out in measurement cells comprising a porous barrier element that define a measurement chamber where the rock sample is received. The porous barrier element is generally a disk located downstream the rock sample, that is permeable to a first fluid, e.g. water or brine, and impervious to a second fluid, e.g. oil.

In a known protocol, the rock sample is prepared by vacuum saturating it with brine, and is then saturated with crude oil in a measurement cell. An imbibition capillary pressure is then measured after an ageing period of the sample saturated with crude oil in an oven.

To achieve this step, the rock sample is removed from the rock measurement cell to achieve the ageing. This leads to depressurizing the sample.

Thus, the known method is not entirely satisfactory. Changes of conditions to get to ambient conditions may be associated with a change of fluid distribution and thus to different experimental conditions than those prevailing at saturation under pressure.

Moreover, handling the sample outside of the measurement cell can be tedious and can raise safety issues.

Finally, when the sample is imbibed after aging, only the negative part of the imbibition capillary pressure is available, as the experiments starts when the sample is not under pressure.

One aim of the invention is thus to provide a rock properties measurement cell that allows a precise and full determination of the rock properties, in particular a full capillary pressure curve cycle, with an easy handling, while minimizing sample disruptions.

To that aim, the invention relates to a rock properties measurement cell characterized by an upstream porous barrier element, impervious to the first fluid and permeable to the second fluid, the upstream porous barrier element defining an downstream face configured to cooperate with a upstream face of the rock sample, an upstream face opposed to the downstream face, and a upstream bypass connecting the upstream face and the downstream face of the upstream porous barrier element;
the upstream diffuser comprising:
   - a first upstream opening, delimiting, together with the upstream bypass, a first upstream channel configured to retrieve first fluid and/or second fluid from the rock sample or to inject first fluid and/or second fluid in the rock sample via the upstream bypass,
   - a second upstream opening forming a second upstream channel, opening facing the upstream face of the upstream porous barrier element and being configured to retrieve only second fluid from the rock sample or to inject only second fluid in the rock sample,
the upstream diffuser, the upstream porous barrier element, the downstream porous barrier element, the downstream diffuser and the sleeve surrounding them delimiting a measurement chamber configured to receive the rock sample.

The rock properties measurement cell may further comprise one or more of the following feature(s), taken separately or according to any technically feasible combination:
- the upstream porous barrier element comprises:
   - a downstream segment of a first diameter, the downstream segment defining the downstream face configured to cooperate with the rock sample,
   - an upstream segment of a second diameter smaller than the first diameter, the upstream segment and the downstream segment defining together the upstream face opposed to the downstream face, the upstream bypass connecting the upstream face and the downstream face of the upstream porous barrier element through the upstream segment and the downstream segment;
- the upstream diffuser defines an upstream cavity, the upstream segment of the upstream porous barrier element being received in the upstream cavity, the measurement cell comprising an upstream sealing gasket arranged around the upstream segment of the upstream porous barrier element between the upstream segment and the upstream diffuser;
- the downstream porous barrier element comprises:
   - an upstream segment of a first diameter, the upstream segment defining the upstream face configured to cooperate with a downstream face of the rock sample
   - a downstream segment of a second diameter smaller than the first diameter, the upstream segment and the downstream segment defining together the downstream face opposed to the upstream face,
   the downstream bypass connecting the upstream face and the downstream face of the downstream porous barrier element through the upstream segment and through the downstream segment of the downstream porous barrier element, the upstream segment of the upstream porous barrier element and the downstream segment of the downstream porous barrier element protruding in opposed longitudinal directions of the rock properties measurement cell;
- the wettability of the downstream porous barrier element to the first fluid is greater than the wettability of the upstream porous barrier element to the first fluid, the wettability of the downstream porous barrier element to the second fluid is smaller than the wettability of the upstream porous barrier element to the second fluid.

The invention also relates to a rock properties measurement system comprising a rock properties measurement cell as defined above and a fluid distribution and collection device connected to the measurement cell, configured to selectively feed the first fluid and the second fluid to the rock properties measurement cell.

The rock properties measurement system may further comprise one or more of the following feature(s), taken separately or according to any technically feasible combination:
- a first upstream valve, fluidly connected to the first upstream opening, and configured to be actuated between an open position freeing fluid passage through the first upstream opening to feed first fluid and/or second fluid to the first upstream channel or retrieve first fluid and/or second fluid from the first upstream channel, and a closed position, blocking fluid passage through the first upstream opening;
- a second upstream valve, fluidly connected to the second upstream opening, and configured to be actuated between an open position freeing fluid passage through the second upstream opening to feed second fluid to the second upstream channel or retrieve second fluid from the second upstream channel, and a closed position, blocking fluid passage through the second upstream opening;
- a first downstream valve, fluidly connected to the first downstream opening, and configured to be actuated between an open position freeing fluid passage through the first downstream opening to feed first fluid and/or second fluid to the first downstream channel or retrieve first fluid and/or second fluid from the first downstream channel, and a closed position, blocking fluid passage through the first downstream opening ;
- a second downstream valve, fluidly connected to the second downstream opening, and configured to be actuated between an open position freeing fluid passage through the second downstream opening to feed first fluid to the second downstream channel or retrieve first fluid from the second downstream channel, and a closed position, blocking fluid passage through the second upstream opening;
- a pressure difference measuring system configured to be selectively connected between (i) at least an upstream pressure measurement point representative of the pressure in one of the first upstream channel and of the second upstream channel, (ii) at least a downstream pressure measurement point representative of the pressure in one of the first downstream channel and of the second downstream channel.
- an inner structure fluid imaging device arranged at an outside of the rock properties measurement cell, wherein the inner structure imaging device is advantageously a microtomography imaging device or nuclear magnetic resonance imaging device.
- a first fluid or second fluid pressurized feeder, configured to be selectively connected to the first upstream channel and/or to the second upstream channel, and comprising a first fluid or second fluid pressurized feeder, configured to be selectively connected to the first downstream channel and/or to the second downstream channel.

The invention additionally relates to a rock properties measurement method, comprising:
- providing a rock sample containing a saturation of a second fluid in a rock properties measurement cell of a rock properties measurement system as defined above, the downstream face of the upstream porous barrier element being applied on an upstream face of the rock sample, the upstream face of the downstream porous barrier element being applied on an downstream face of the rock sample ;
- injecting first fluid in the rock sample through the first downstream channel, and recovering second fluid through the second upstream channel, the first upstream channel being blocked to fluid passage, the second upstream channel being advantageously connected to a second fluid constant pressure reservoir;
- during or/and after the injecting of first fluid in the rock sample through the first downstream channel, measuring at least a rock sample property, in particular at least one of a capillary pressure in the rock sample during and/or after injection of the first fluid and a fluid saturation in the rock sample during and/or after injection of the first fluid.

The rock properties measurement method may further comprise one or more of the following feature(s), taken separately or according to any technically feasible combination:
- before injecting first fluid in the rock sample through the first downstream channel, flushing second fluid from the second upstream channel through the upstream porous barrier element to the first upstream channel, the injecting of first fluid in the rock sample through the first downstream channel being carried out after the flushing of second fluid, without disconnecting or moving the rock properties measurement cell from the fluid distribution and collection device of the rock properties measurement system;
- providing a rock sample containing a saturation of a second fluid in a rock properties measurement cell comprises:
   * draining second fluid in the rock sample containing first fluid, through the first upstream channel, and recovering first fluid through the second downstream channel, the first downstream channel being blocked to fluid passage, the second downstream channel being advantageously connected to a first fluid constant pressure reservoir, in order to reach a target first fluid saturation and/or capillary pressure, and measuring at least a rock sample property, in particular at least one of a capillary pressure in the rock sample during and/or after draining of the second fluid and a fluid saturation in the rock sample during and/or after draining of the second fluid and/or :
   * imbibing first fluid in the rock sample containing second fluid, through the second downstream channel, the second downstream channel being advantageously connected to a first fluid constant pressure reservoir and recovering first fluid and/or second fluid through the first upstream channel, measuring at least a rock sample property, in particular at least one of a capillary pressure in the rock sample during and/or after imbibition of the first fluid and a fluid saturation in the rock sample during and/or after imbibition of the first fluid;
   the injecting of first fluid in the rock sample through the first downstream channel being carried out after the imbibing of first fluid, without disconnecting or moving the rock properties measurement cell from the fluid distribution and collection device of the rock properties measurement system;
- after draining second fluid in the rock sample, placing the rock properties measurement cell in an oven to heat the rock properties measurement cell while keeping connections with the fluid distribution and collection device and maintaining the target first fluid saturation and/or capillary pressure;
- the first fluid is water, the second fluid being oil.

Other features and advantages of the invention will become apparent from the following detailed description, which is given purely as an example and by no means as a limitation, made in reference to the appended drawings, in which:
- Figure 1 is a schematic representation of a rock properties measurement system comprising a rock properties measurement cell according to the invention;
- Figure 2 is a schematic perspective view of an upstream porous barrier element and of a downstream monolithic porous barrier element in the rock properties measurement cell of figure 1;
- Figure 3 is a view similar to figure 1, during a primary flooding of a second fluid implemented in the rock properties measurement cell;
- Figure 4 is a curve of the capillary pressure as a function of the saturation in first fluid during a primary flooding of second fluid implemented in figure 3;

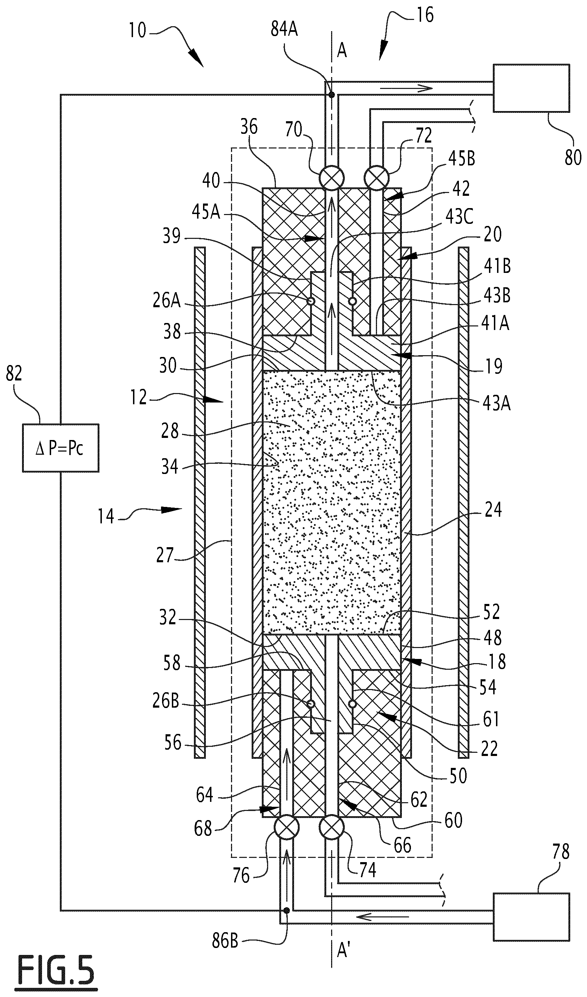

- Figure 5 is a view similar to figure 1, during a first fluid imbibition implemented in the rock properties measurement cell, after the primary flooding of figure 3;
- Figure 6 is a curve of the capillary pressure as a function of the saturation in first fluid during the first fluid imbibition implemented in figure 5;
- Figure 7 is a view similar to figure 1, during a flushing with second fluid of the upstream porous barrier element, implemented in the rock properties measurement cell after the first fluid imbibition of figure 6;
- Figure 8 is a view similar to figure 1, during a first fluid injection implemented in the rock properties measurement cell to measure a negative capillary pressure curve after the second fluid flushing of figure 7;
- Figure 9 is a curve of the capillary pressure as a function of the saturation in first fluid injection during the first fluid injection implemented in figure 8.

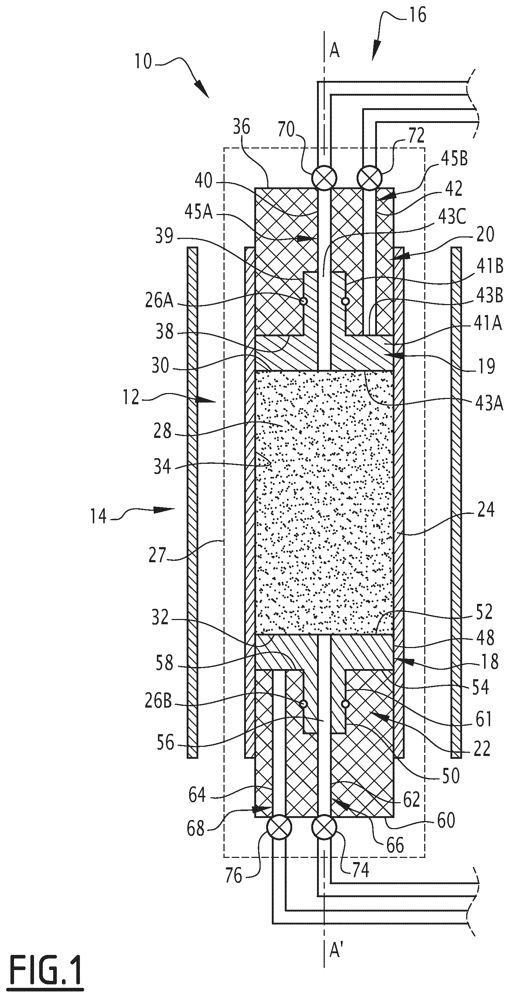

Referring to figure 1, a rock properties measurement system 10 according to the invention comprises a rock properties measurement cell 12 and advantageously, an inner structure imaging device 14 configured to image a rock sample 28 placed in the rock properties measurement cell 12 and optionally fluid(s) contained in the rock sample.

When no imaging device is available, the rock properties measurement system 10 may comprise a mass balance measurement system, e.g. to determine average saturations in fluids F1, F2.

The rock properties measurement system 10 also contains a fluid distribution and collection device 16 connected to the rock properties measurement cell 12 to selectively inject at least a fluid F1, F2 in the rock properties measurement cell 12 or/and to selectively recover at least a fluid F1, F2 from the rock properties measurement cell 12.

The rock properties measurement cell 12 extends longitudinally along an axis A-A' between an upstream end at the top of the rock properties measurement cell 12 in figure 1 and a downstream end at the bottom of the rock properties measurement cell 12 in figure 1.

It comprises a downstream porous barrier element 18 configured to contact a downstream face 32 of the rock sample 28 received in the rock properties measurement cell 12 and a downstream diffuser 22, placed in contact with the downstream porous barrier element 18 opposite the rock sample 28.

According to the invention, the rock properties measurement cell 12 comprises an upstream porous barrier element 19 configured to contact an upstream face 30 of the rock sample 28 received in the rock properties measurement cell 12 and an upstream diffuser 20, placed in contact with the upstream porous barrier element 19, opposite the rock sample 28.

The rock properties measurement cell 12 further comprises a sleeve 24 surrounding and containing the downstream porous barrier element 18, the downstream diffuser 22, the upstream porous barrier element 19, and the upstream diffuser 20.

The rock properties measurement cell 12 further comprises respective upstream and downstream sealing gaskets 26A, 26B interposed respectively between the upstream porous barrier element 19 and the upstream diffuser 20 and between the downstream porous barrier element 18 and the downstream diffuser 22.

It advantageously comprises a confinement enclosure 27 to seal the upstream diffuser 20 and the downstream diffuser 22.

The rock properties measurement cell 12 is configured to receive the rock sample 28 to carry out for example a cycle of capillary pressure measurements, preferably associated with inner structure imaging of at least a fluid F1, F2, contained in said rock sample 28.

In the following description, the terms "upstream" and "downstream" are understood by reference with the displacement of a first fluid F1, under the injection of a second fluid F2, within the rock properties measurement cell 12 during a primary drainage of the first fluid F1 by the second fluid F2. In figure 1, an upstream element or surface is located higher than a downstream element or surface.

As this will be exposed later, the first fluid F1 is preferably water and the second fluid is preferably oil or gas.

In particular, the first fluid is preferably water including at least one salt, such as a brine, with a salt concentration comprised between 1 g/l and 300 g/l. The water including at least one salt may comprise a plurality of salts of different compositions.

The second fluid is for example an oil or a gas chosen from the following types of oils and gas: mineral synthetic oil, dead oil, live oil, Nitrogen, compressed air, Helium, H₂, CO₂ or other gas mixtures.

In alternative, and for example, the first fluid F1 is oil and/or the second fluid F2 is water.

It will be understood that the first fluid F1 and/or the second F2 fluid can also be other fluids differing from water and oil.

As seen on figure 1, the upstream diffuser 20, the upstream porous barrier element 19, the rock sample 28, the downstream porous barrier element 18 and the downstream diffuser 22 are aligned in this order in a flow direction of the at least one fluid F1, F2 along the longitudinal axis A-A'.

The rock sample 28 is of any type, e.g. homogeneous or heterogeneous. It is for example a rock sample 28 that is extracted from an oil and gas exploration site. The permeability of rock sample 28 can take values below 1mD (millidarcy) up to several Darcies (for example up to 10 Darcies).

The rock sample 28 defines a rock sample axis which is coaxial to the longitudinal axis A-A' when the rock sample 28 is received in the rock properties measurement cell 12. The rock sample 28 is preferably elongated along the longitudinal axis A-A'.

The rock sample 28 comprises an upstream face 30 and a downstream face 32. The upstream face 30 and the downstream face 32 are opposed relatively to the longitudinal axis A-A' and are transverse to the longitudinal axis A-A'.

In the example of figure 1, the rock sample 28 is cylindrical.

A length L of the rock sample 28 along the longitudinal axis A-A' is for example comprised between 5 mm and 500 mm.

The rock sample is preferably cylindrical. A diameter W of the rock sample 28, taken transversally to the longitudinal axis A-A', is for example comprised between 10 mm and 75 mm.

As illustrated in figure 1, the rock sample 28 is received in a measurement chamber 34 of the measurement cell 12.

The measurement chamber 34 is delimited axially by the upstream porous barrier element 19 in contact with the upstream face 30 of the rock sample 28, and by the downstream porous barrier element 18 in contact with the downstream face 32 of the rock sample 28. The measurement chamber 34 is delimited laterally by the sleeve 24.

The dimension of the measurement chamber 34 is for example slightly smaller than the dimensions of the rock sample 28. The rock sample 28 is thus for example in contact with the sleeve 24 when the rock sample 28 is received in the measurement chamber 34.

The sleeve 24 is arranged around the upstream diffuser 20, the upstream porous barrier element 19, the downstream diffuser 22 and the downstream porous barrier element 18.

As illustrated in figure 1, the sleeve 24 is arranged around the downstream porous barrier element 18 to surround the entire downstream porous barrier element 18 along the longitudinal axis A-A' and is arranged to at least partially surround the downstream diffuser 22.

The sleeve 24 is arranged around the upstream porous barrier element 19 to surround the entire upstream porous barrier element 19 along the longitudinal axis A-A' and is arranged to at least partially surround the upstream diffuser 20.

The sleeve 24 is preferably arranged to surround the rock sample 28 along the longitudinal axis A-A'. In the example of figure 1 where the rock sample 28 is cylindrical, the sleeve 24 is a tubular.

The inner diameter of the sleeve 24 therefore substantially corresponds to the width W of the rock sample 28, the inner diameter of the sleeve 24 being inferior or equal to the width W of the rock sample 28.

For example, the inner diameter of the sleeve 24 is equal to 8 mm when the width W of the rock sample 28 is equal to 10 mm and is equal to 36 mm when the width W of the rock sample 28 is equal to 38 mm, the elasticity of the sleeve 24 allowing the sleeve to be arranged around the rock sample 28.

As seen on figure 1, the sleeve 24 preferably protrudes on both sides of the rock sample 28 along the longitudinal axis A-A' so that is also surround at least a partially the upstream diffuser 20 and the downstream diffuser 22.

A thickness of the sleeve 24 is for example equal to 1 mm.

The sleeve 24 is transparent to inner structure imaging such as microtomography imaging and/or nuclear magnetic resonance imaging.

The sleeve 24 is impervious to the fluids present in the rock sample F1, F2 so that the sleeve 24 guides the fluids F1 and/or F2 along the longitudinal axis A-A' within the rock sample 28.

The sleeve 24 is for example made of a polyketone, such as a polyaryletherketone, in particular polyether ether ketone (PEEK) or of a fluorocarbon-based fluoroelastomer. The sleeve 24 is for example made of Viton^{®}, a fluorocarbon-based fluoroelastomer developed by the company Viton^{™}.

As it will be presented later, the sleeve 24 is for example pressed around the upstream diffuser 20, the downstream diffuser 22, the upstream porous barrier element 19, the downstream porous barrier element 18 and the rock sample 28 received in the measurement chamber 34 when the measurement cell 12 is used to measure rock properties.

The upstream diffuser 20 is aligned to the longitudinal axis and is located upstream of the measurement chamber 34, or in other terms, upstream of the rock sample 28.

The upstream diffuser 20 is preferably transparent to inner structure imaging such as microtomography imaging and/or nuclear magnetic resonance imaging.

The upstream diffuser 20 is for example made of a poly ketone polymer, in particular polyether ether ketone.

The upstream diffuser 20 is configured to guide the first fluid and the second fluid to the rock sample 28 and from the rock sample 28 through the upstream porous barrier element 19.

The upstream diffuser 20 comprises an upstream side 36 and a downstream side 38 opposed to the upstream side 36 and in contact with the upstream porous barrier element 19.

The upstream side 36 of the upstream diffuser 20 is for example essentially flat.

As illustrated in figure 1, the downstream side 38 defines an upstream cavity 39.

The upstream cavity 39 is delimited by two annular sub-faces of the downstream side 38 connected together by a cylindrical sub-face of the downstream side 38.

The cavity 39 is configured to receive at least an upstream segment 41B of the upstream porous barrier element 19. The shape of the cavity 39 is complementary to the shape of the upstream segment 41B.

The upstream diffuser 20 also comprises a first upstream opening 40 and a second upstream opening 42.

The first upstream opening 40 extends through the upstream diffuser 20 to connect the downstream side 38 and the upstream side 36 of the upstream diffuser 20, in the upstream cavity 39.

The first upstream opening 40 is configured to guide first fluid F1 or/and second fluid F2 towards the rock sample 28 from the upstream side 36 to the downstream side 38 of the upstream diffuser 20 or first fluid F1 or/and second fluid F2 from the rock sample 28, from the downstream side 38 to the upstream side 36 of the upstream diffuser 20.

The second upstream opening 42 extends through the upstream diffuser 20 to connect the downstream side 38 and the upstream side 36 of the upstream diffuser 20, apart from the cavity 39. The second upstream opening 42 is configured to guide only second fluid F2 from the upstream side 36 to the downstream side 38 of the upstream diffuser 20 to the upstream porous barrier element 19 or from the upstream porous barrier element 19 from the downstream side 38 to the upstream side 36 of the upstream diffuser 20.

According to the invention, the upstream porous barrier element 19 is aligned to the longitudinal axis A-A' and is located upstream of the measurement chamber 34 containing the rock sample 28.

The upstream porous barrier element 19 is for example made of ceramic and for example an alumina ceramic Al₂O₃ made oil-wet. Oil-wet ceramics are for example manufactured by COBRA company. It is advantageously made in one piece.

Pores size of the upstream porous barrier element 19 is for example comprised between 20 nm and 200 nm and is for example equal to 50 nm, 80 nm or 150 nm.

The upstream porous barrier element 19 is permeable to the second fluid F2 and is impervious to the first fluid F1 in presence of the second fluid F2. In other words, when a first fluid F1 and a second fluid F2 are located within the rock sample 28 and the second fluid F2 is connected from the upstream porous barrier element 19 to the upstream segment 45B, only the second fluid F2 can be retrieved from the rock sample 28 through the porous network of the upstream porous barrier element 19.

The wettability of the upstream porous barrier element 19 to the second fluid F2 is greater than the wettability of the upstream porous barrier element 19 to the first fluid F1. The wetting of the upstream porous barrier 19 is for example measured with the contact angle measurement method on a surface 43A, 43B of the upstream porous barrier element 19. The method consists in saturating the upstream porous barrier 19 with first fluid F1 and immersing the saturated upstream porous barrier 19 in fluid F1. After then, a droplet of second fluid F2 is put on surface 43A or 43B. If the droplet draws a contact angle greater than 90° with the surface, then the upstream porous barrier 19 is wetting to first fluid F1. If not, the upstream porous barrier 19 is wetting to second fluid F2.

The contact angle of the second fluid F2 on the surface 43A and 43B, in presence of the first fluid F1 is for example greater than 90°.

The particular wettability of the upstream porous barrier element 19 is obtained from the nature of the material forming the upstream porous barrier element 19, or/and from a coating applied of the ceramic to enhance the affinity to the second fluid F2.

The upstream porous barrier element 19 comprises a downstream segment 41A of a first diameter D1 and an upstream segment 41B of a second diameter D2. The second diameter D2 is smaller than the first diameter D1. In a median cross section, the upstream segment 41B and the downstream segment 41A define an inverted T shape of the upstream porous barrier element 19.

The downstream segment 41A defines a downstream contact face 43A with the rock sample 28. As visible on figure 1, the downstream contact face 43A is in contact with the upstream face 30 of the rock sample 28.

As visible on figure 1, the downstream contact face 43A is for example essentially flat.

The upstream segment 41B and the downstream segment 41A define together an upstream face 43B opposed to the downstream face 43A. As shown on figure 1, the upstream face 43B therefore comprise two annular sub-faces connected together by a cylindrical sub-face.

The upstream porous barrier element 19 comprises an upstream bypass 43C connecting the upstream face 43B and the downstream face 43A through both the upstream segment 41B and the downstream segment 41A. Thus, when a first fluid F1 and a second fluid F2 are located within the rock sample 28, both the first fluid F1 and the second fluid F2 can be retrieved from the rock sample 28 through the upstream bypass 43C.

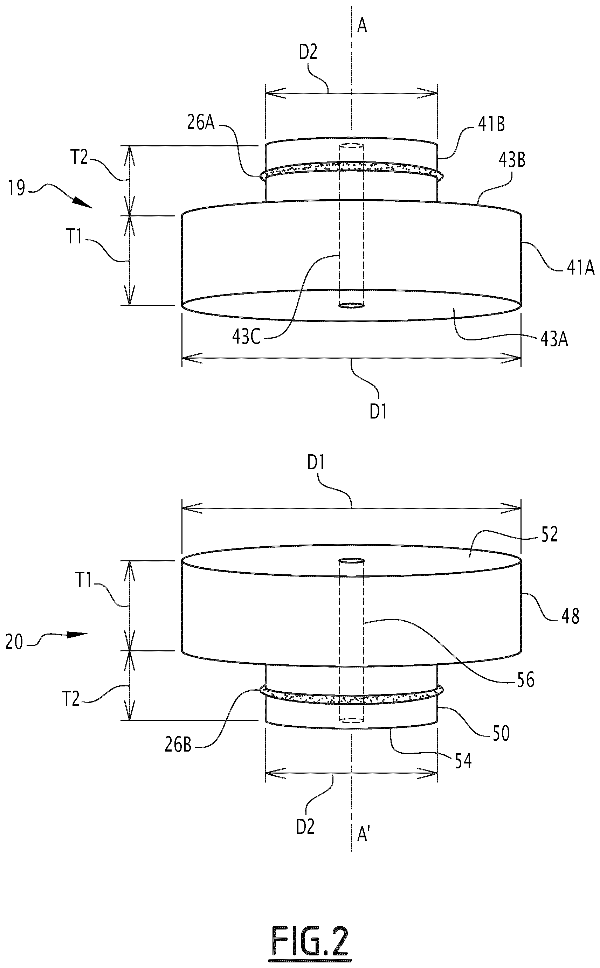

In the example presented in figure 2, the upstream porous barrier element 19 is a revolution element and the bypass 43C extends through the upstream porous barrier element 19 following the axis of revolution of the element. In particular, the bypass 43C is aligned to the longitudinal axis A-A'.

The first diameter D1 preferably corresponds to the width W of the rock sample 28. The thickness T1 of the downstream segment 41A is preferably comprised between 3 mm and 20 mm.

The ratio of the thickness T1 over the diameter D1 of the downstream segment 41A is for example smaller than 40%. The downstream segment 41A is disk shaped.

The thickness T2 of the upstream segment 41B is preferably comprised between 5 mm and 50 mm.

The upstream segment 41B is received in the upstream cavity 39 in a complementary manner. The upstream sealing gasket 26A is interposed between the upstream segment 41B and the upstream diffuser 20 in the upstream cavity 39 to seal the interface between the upstream porous barrier element 19 and the upstream diffuser 20.

The first upstream opening 40 is aligned with the upstream bypass 43C, that is, the first upstream opening 40 is fluidly connected to the upstream bypass 43C. The first upstream opening 40 delimits together with the bypass 43C a first upstream open channel 45A.

The first upstream channel 45A opens directly on the rock sample 28 and extends through the upstream diffuser 20 and the upstream porous barrier element 19.

The first upstream channel 45A is configured to inject first fluid F1 and/or second fluid F2 in the rock sample 28 and to retrieve first fluid F1 and/or second fluid F2 from the rock sample 28.

The second upstream opening 42 forms a second upstream open channel 45B.

The second upstream open channel 45B opens facing the upstream porous barrier element 19 and in particular facing the downstream segment 41A on the upstream face 43B.

The second upstream open channel 45B is configured to inject or retrieve second fluid F2 from the rock sample 28. In particular, the second upstream open channel 45B is configured to retrieve only the second fluid F2 from the rock sample 28 that passed through the upstream porous barrier element 19.

The downstream porous barrier element 18 is aligned to the longitudinal axis A-A' and is located downstream of the measurement chamber 34 containing the rock sample 28.

The downstream porous barrier element 18 is for example made of ceramic and for example an alumina ceramic Al₂O₃. This downstream porous barrier element is preferentially wetting to first fluid F1 rather than to second fluid F2. It is advantageously made in one piece.

Pores size of the downstream porous barrier element 18 is for example comprised between 20 nm and 200 nm and is for example equal to 50 nm, 80 nm or 150 nm.

The downstream porous barrier element 18 is permeable to the first fluid F1 in presence of the second fluid F2 and impervious to the second fluid F2 in presence of the first fluid F1. In other words, when a first fluid F1 and a second fluid F2 are located within the rock sample 28, only the first fluid F1 can be retrieved from the rock sample 28 through the porous network of the porous barrier element 18.

The wettability of the downstream porous barrier element 18 to the first fluid F1 is greater than the wettability of the upstream porous barrier element 19 to the first fluid F1. The wettability is for example measured with a contact angle measurement on a surface 52, 54 of the upstream porous barrier element 19, following the standard contact angle measurement methodology in presence of first fluid F1 and second fluid F2.

The contact angle of the first fluid F1 on the surface 52, 54 is for example lower than 90°.

The wettability of the downstream porous barrier element 18 to the first fluid F1 is greater, in particular with contact angles lower than 60°, which is lower than the first fluid contact angles obtained on the upstream porous barrier element 19 to the first fluid F1 which are greater than 90°. On the contrary, the wettability of the downstream porous barrier element 18 to the second fluid F2 is smaller, in particular with contact angles greater than 90°, than the wettability of the upstream porous barrier element 19 to the second fluid F2 with contact angles lower than 70°.

The downstream porous barrier element 18 comprises an upstream segment 48 of a first diameter D1 and a downstream segment 50 of a second diameter D2. The second diameter D2 is smaller than the first diameter D1. In a median cross section, the upstream segment 48 and the downstream segment 50 define a T shape of the downstream porous barrier element 18.

The upstream segment 48 defines an upstream face 52 in contact with a downstream face 32 of the rock sample 28.

As visible on figure 1, the upstream face 52 is for example essentially flat.

The upstream 48 and downstream 50 segments define together a downstream face 54 opposed to the upstream face 52. As shown on figure 2, the downstream face 54 therefore comprise two annular sub-faces connected together by a cylindrical sub-face.

The porous barrier element 18 comprises a downstream bypass 56 connecting the upstream face 52 and the downstream face 54 through both the upstream segment 48 and the downstream segment 50. Thus, when a first fluid F1 and a second fluid F2 are located within the rock sample 28, both the first fluid F1 and the second fluid F2 can be retrieved from the rock sample 28 through the downstream bypass 56.

In the example presented in figure 2, the porous barrier element 18 is a revolution element and the downstream bypass 56 extends through the porous barrier element 18 following the axis of revolution of the element. In particular, the downstream bypass 56 is aligned to the longitudinal axis A-A'.

The first diameter D1 corresponds to the width W of the rock sample 28 The thickness T1 of the upstream segment is preferably comprised between 3 mm and 20 mm.

The ratio of the thickness T1 over the diameter D1 of the upstream segment 48 is for example smaller than 40%. The upstream segment 48 is disk shaped.

The thickness T2 of the downstream segment 50 is preferably comprised between 5 mm and 50 mm.

In reference to figure 1, the downstream diffuser 22 is aligned to the longitudinal axis A-A' and is located downstream of the downstream porous barrier element 18.

The downstream diffuser 22 is preferably transparent to inner structure imaging such as microtomography imaging and/or nuclear magnetic resonance imaging.

The downstream diffuser 22 is for example made of a poly ketone polymer, in particular polyether ether ketone.

The downstream diffuser 22 comprises an upstream side 58 and a downstream side 60 opposed to the upstream side 56 relatively to the downstream diffuser 22.

The downstream side 60 of the downstream diffuser 22 is for example essentially flat.

As illustrated in figure 1, the downstream diffuser 22 defines a downstream cavity 61.

The downstream cavity 61 is delimited by the upstream side 58 of the downstream diffuser 22. As shown on figure 1, the upstream side 58 therefore comprise two annular sub-faces connected together by a cylindrical sub-face.

The downstream cavity 61 is conformed to receive the downstream porous barrier element 18. In particular, and as shown on figure 1, the downstream segment 50 of the porous element 18 is received in the downstream cavity 61, the shape of the downstream cavity 61 being complementary to the shape of the downstream segment 50 of the porous element 18.

The downstream sealing gasket 26B is interposed between the dowstream segment 50 and the downstream diffuser 22 in the downstream cavity 61 to seal the interface between the downstream porous barrier element 18 and the downstream diffuser 22.

The downstream diffuser 22 comprises a first downstream opening 62 and a second downstream opening 64.

The first downstream opening 62 is aligned with the downstream bypass 56, that is, the first downstream opening 62 is connected to the bypass 56. The first downstream opening 62 delimits together with the bypass 56 a first downstream open channel 66.

The first open channel 66 opens directly on the rock sample 28 and extends through the downstream diffuser 22 and the downstream porous barrier element 18.

The first open channel 66 is configured to inject first fluid F1 and/or second fluid F2 in the rock sample 28 and to retrieve first fluid F1 and/or second fluid F2 from the rock sample 28.

The second downstream opening 64 forms a second downstream open channel 68.

The second downstream open channel 68, opens facing the downstream porous barrier element 18 and in particular facing the upstream segment 48 on the downstream face 54.

The second downstream open channel 68 in configured to retrieve first fluid F1 from the rock sample 28. In particular, the second downstream open channel 68 is configured to inject or retrieve only first fluid F1 from the rock sample 28 that passed through the downstream porous barrier element 18.

As illustrated in figure 1, the confinement enclosure 27 is arranged around the sleeve 24. Since the sleeve 24 is arranged at least partially around the upstream diffuser 20, the upstream porous barrier element 19, the downstream diffuser 22 and the downstream porous barrier element 18, the confinement enclosure 27 is also arranged around the upstream diffuser 20, the upstream porous barrier element 19, the downstream diffuser 22 and the downstream porous barrier element 18, and around the rock sample 28 received in the measurement chamber 34.

The confinement enclosure 27 is transparent to inner structure imaging such as 1D, 2D and 3D X-ray imaging devices and/or nuclear magnetic resonance imaging.

The confinement enclosure 27 is for example made of a poly ketone polymer, in particular polyether ether ketone.

The confinement enclosure 27 is configured to receive a confinement fluid to apply pressure to the sleeve 24 around the upstream diffuser 20, the upstream porous barrier element 19, the downstream diffuser 22, the downstream porous barrier element 18 and the rock sample 28 received in the measurement chamber 34.

The confinement fluid CF is transparent to inner structure imaging such as 1D, 2D and 3D X-ray imaging devices and/or nuclear magnetic resonance imaging.

The confinement fluid CF is for example water, mineral oil, nitrogen and for example a fluorinated oil.

The fluid distribution and collection device 16 comprises a first upstream valve 70, a second upstream valve 72, a first downstream valve 74 and a second downstream valve 76. It comprises a pressurized first fluid F1 feeder 78 and a pressurized second fluid F2 feeder 80.

The fluid distribution and collection device 16 also comprises a pressure measuring system 82.

The first upstream valve 70 is fluidly connected to the first upstream opening 40 for example via a connection tube (not shown).

The first upstream valve 70 is configured to selectively free or block the first upstream opening 40. It can be actuated between an open position freeing fluid passage through the first upstream opening 40 to feed first fluid F1 and/or second fluid F2 to the first upstream channel 45A or retrieve first fluid F1 and/or second fluid F2 from the first upstream channel 45A, and a closed position, blocking fluid passage through the first upstream opening 40 and through the first upstream channel 45A.

The second upstream valve 72 is fluidly connected to the second upstream opening 42 for example via a connection tube (not shown).

The second upstream valve 72 is configured to selectively free or block the second upstream opening 42. It can be actuated between an open position freeing fluid passage through the second upstream opening 42 to feed second fluid F2 to the second upstream channel 45B or retrieve second fluid F2 from the second upstream channel 45B, and a closed position blocking fluid passage through the second upstream opening 42 and the second upstream channel 45B.

The first downstream valve 74 is fluidly connected to the first downstream opening 62 for example via a connection tube (not shown).

The first downstream valve 74 is configured to selectively free or block the first downstream opening 62. It can be actuated between an open position freeing fluid passage through the first downstream opening 62 to feed first fluid F1 or second fluid F2 to the first upstream channel 66 or retrieve first fluid F1 and/or second fluid F2 from the first downstream channel 66, and a closed position, blocking fluid passage through the first downstream opening 62 and the first downstream channel 66.

The second downstream valve 76 is fluidly connected to the second downstream opening 64 for example via a connection tube (not shown).

The second downstream valve 76 is configured to selectively free or block the second downstream opening 64. It can be actuated between an open position freeing fluid passage through the second downstream opening 64 to feed first fluid F1 to the second downstream channel 66 or retrieve first fluid F1 from the second downstream channel 66, and a closed position, blocking fluid passage through the second upstream opening 64 and the second downstream channel 66.

The first fluid feeder 78 is configured to be connected to the first downstream valve 74 to feed first fluid F1 to the downstream face 32 of the rock sample 28 through the first downstream channel 66.

In the embodiment presented in figures 1 and 2, the first fluid feeder 78 comprises for example a pump.

As it will be presented later, the first fluid feeder 78 is configured for either feeding the first fluid F1 with a constant flow, or for feeding first fluid F1 with a constant pressure.

When the first fluid feeder 78 is configured to feed the first fluid F1 with a constant pressure, it is for example logically connected with the pressure measuring system 82, the first fluid feeder 78 feeding the first fluid F1 at a flowrate so that the pressure measured by the pressure measuring system 82 is constant.

The second fluid feeder 80 is configured to be connected to the first upstream valve 70 to feed first fluid F1 or second fluid F2 to the upstream face 30 of the rock sample 28 through the first upstream channel 45A.

In the embodiment presented in figures 1 and 2, the second fluid feeder 80 comprises for example a pump.

As it will be presented later, the second fluid feeder 80 is configured for either feeding the second fluid F2 with a constant flow, or for feeding second fluid F2 with a constant pressure.

When the second fluid feeder 80 is configured to feed the second fluid F2 with a constant pressure, it is for example logically connected with the pressure measuring system 82, the second fluid feeder 80 feeding the second fluid F2 at a flowrate so that the pressure measured by the pressure measuring system 82 is constant.

The pressure measuring system 82 comprises a first upstream pressure measurement point 84A in the first upstream channel 45A or fluidly connected to the first upstream channel 45A, and a second upstream pressure measurement point 84B in the second upstream channel 45B or fluidly connected to the second downstream channel 45B.

The pressure measuring system 82 further comprises a first downstream pressure measurement point 86A in the first downstream channel 66 or fluidly connected to the first downstream channel 66, and a second downstream pressure measurement point 86B in the second downstream channel 68 or fluidly connected to the second downstream channel 68.

It comprises a computer configured to collect data measured at the measurement points 84A, 84B, 86A, 86B and to determine at least an information representative of the capillary pressure Pc or pressure gradient in the rock sample 28 from a difference between data at measurement points 84A, 84B and 86A, 86B,

As illustrated in figure 1, the inner structure fluid imaging device 14 is arranged at an outside of the rock properties measurement cell 12.

The inner structure fluid imaging device 14 is for example arranged around the confinement enclosure 27.

The inner structure fluid imaging device 14 is configured to evaluate fluids saturation in first fluid F1 and/or second fluid F2 along the longitudinal axis A-A'.

The inner structure imaging device 14 is for example a 1D or 2D or 3D x-ray imaging device or nuclear magnetic resonance imaging device.

A rock property measurement method carried out with the rock properties measurement cell 12 of the rock properties measurement system 10 according to the invention will now be described.

In the example, primary drainage and first imbibition capillary pressure curves are determined using the method according to the invention. This information helps understanding an initial state of a petroleum reservoir and predicting oil recovery during a waterflooding process.

Primary drainage capillary pressure curve mimics the first oil migration in the reservoir when the rock was initially having high affinity with underground water (water wet). Then, during millions of years, chemical interactions occurred between the fluids and the rock and altered the reservoir wettability until its discovery. The modified wettability plays a major role in fluid flow in the reservoir pore network.

When injecting extra brine in the reservoir to push and recover oil from the reservoir (waterflooding process), imbibition capillary pressure curve plays in big role in predicting oil recovery as a function of time.

Initially, the dry rock sample 28 is inserted between the downstream porous barrier element 18 which is placed in contact with a downstream face 32 of the rock sample 28 and the upstream porous barrier element 19, which is placed in contact with an upstream face 30 of the rock sample 28.

The downstream diffuser 22 is positioned in contact with the downstream porous barrier element 18 opposite the rock sample 28.

The upstream diffuser 20 is positioned in contact with the upstream porous barrier element 19 opposite the rock sample 28.

The sleeve 24 is then mounted to surround and contain the downstream diffuser 22, the downstream porous barrier element 18, the rock sample 28, the upstream porous barrier element 19, and the upstream diffuser 20.

A confining pressure is applied to the sleeve 24 surrounding the rock sample 28 to ensure that fluid flow occurs through the porous network system of the rock sample 28.

Initially, the first upstream valve 70 and the first downstream valve 74 are closed. Vacuum is applied to the dry rock sample 28 and to the porous barriers 18 and 19 through the upstream second valve 72 and downstream second valve 76. One of these two valves 72, 76 is connected to a vacuum device and the other one is connected to an absolute pressure sensor to measure the vacuum.

When the pressure sensor reaches a vacuum pressure of at least -0.996 bars relative, the vacuum is considered to be adequate. All valves 70, 72, 74, 76 are closed.

Then, first fluid F1 (e.g. water) is introduced through the first downstream valve 74 via the downstream bypass 56 and circulates towards the first upstream valve 70 via the upstream bypass 43C and also fills the rock sample 28, the upstream and downstream porous barriers 18 and 19 and channels 64 and 42.

The rock sample 28 and the whole measurement chamber 34 inside the cell 22 are then saturated with first fluid F1. The saturation in first fluid F1 is then equal to 1.

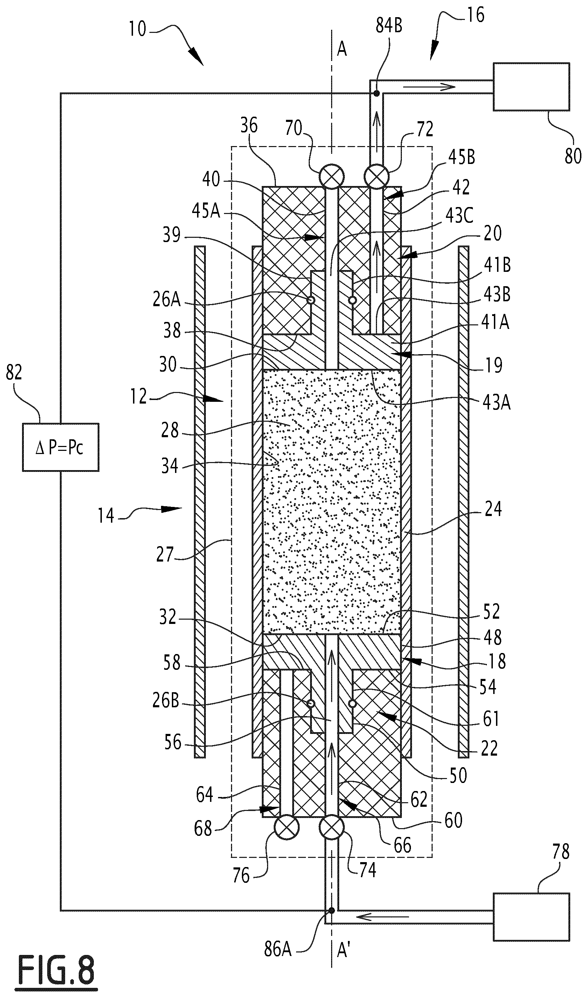

Multiple flowrates of the first fluid F1 introduced in the rock sample 28 are adjusted to be constant and the pressure differences between the first upstream measurement point 84A and the first downstream measurement point 86A (see figure 8) is measured to obtain permeability of the rock sample 28 via Darcy's law.

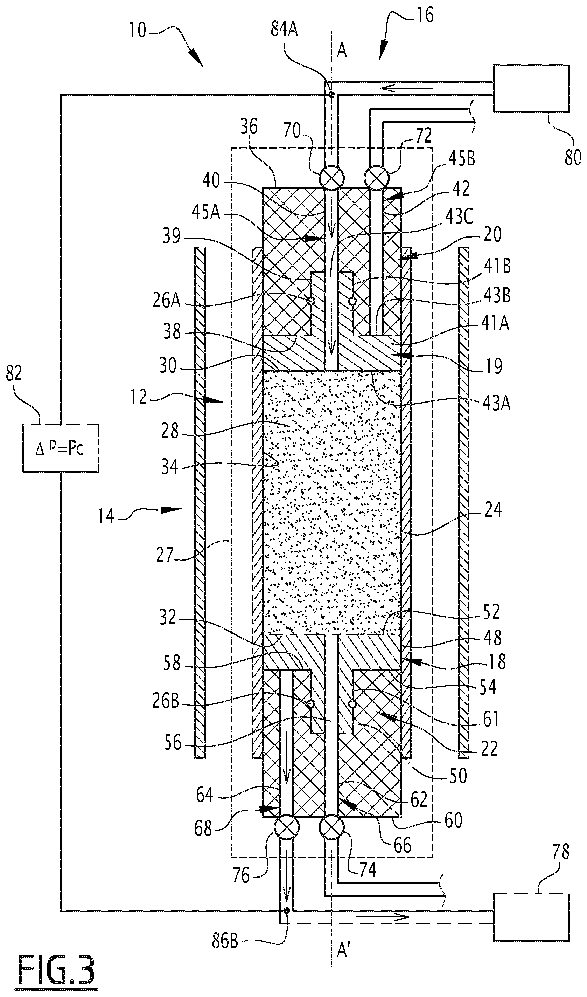

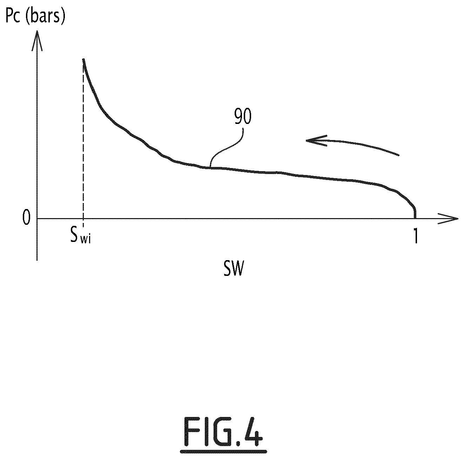

Then, as shown in figures 3 and 4, a primary drainage with second fluid F2 (e.g. oil) is carried out. The second upstream valve 72 and the first downstream valve 74 are closed. The pressurized second fluid feeder 80 is connected to the first upstream channel 45A via the first upstream valve 70 which is opened. The second downstream channel 68 is connected to a constant pressure reservoir of first fluid F1, e.g. in the pressurized first fluid feeder 78, via the second downstream valve 76 which is opened.

Second fluid F2 is injected in the rock sample 28 from the pressurized second fluid feeder 80 via the first upstream channel 45A at various constant pressure steps.

As the rock sample 28 was initially full saturated with first fluid F1, there is a continuity of first fluid F1 between the rock sample 28 and the downstream porous barrier element 18 to the second downstream channel 68.

Thus, the second fluid F2 pushes the first fluid F1 in the rock sample 28 and first fluid F1 only exits the rock sample 28 through the downstream porous barrier element 18 to the second downstream channel 68 and the constant pressure reservoir as shown in figure 3.

The pressure difference between the first upstream measurement point 84A and the second downstream measurement point 86B is representative of an imposed capillary pressure Pc which depends on the pressure provided by the second fluid pressurized feeder 80.

At each capillary pressure Pc step, when no more first fluid F1 is produced in the second downstream channel 68, an equilibrium capillary pressure Pc is reached in the rock sample 28. At this equilibrium, an image of the rock sample 28 is acquired via the inner structure fluid-imaging device 14 to determine an average saturation Sw and advantageously, a saturation profile along the rock sample 28.

Thus, a first segment 90 of the capillary pressure Pc versus first fluid saturation Sw curve is determined with decreasing saturations in first fluid Sw corresponding to increasing capillary pressures Pc shown by the arrow on Figure 4.

After reaching a targeted final first fluid F1 saturation Swi and capillary pressure, the second downstream valve 76 is closed and the first downstream valve 74 is opened. Second fluid F2 continues to be introduced through the first upstream valve 70 via the upstream bypass 43C and circulates towards the first downstream valve 74 via the downstream bypass 56.

Multiple flowrates of second fluid F2 introduced in the rock sample 28 are adjusted to be constant and the pressure differences between the first upstream measurement point 84A and the first downstream measurement point 86A is measured to obtain permeability of the rock sample 28 via Darcy's law.

During this measurement, the pressure differences do not exceed the last capillary pressure Pc obtained during the primary drainage, that is at the final first fluid F1 saturation target, Swi.

After the primary drainage, the rock properties measurement cell 12 is disconnected from the fluid distribution and collection device 16 of the rock properties measurement system 10 without altering the pressure and fluid composition of the rock sample 28 in the rock properties measurement cell 12 obtained after draining the second fluid F2.

The rock properties measurement cell 12 is placed in an oven to heat the rock properties measurement cell 12 while keeping connections with the pressurized first fluid F1 feeder 78 and the pressurized second fluid F2 feeder 80, with the pressure measuring system 82 and with the fluid distribution and collection device 16.

An ageing of the rock sample 28 is then carried out. The rock sample 28 is aged under temperature, e.g. at geological reservoir temperature for several days (e.g. 14 days to 1 month). For example, the temperature is comprised between 80°C and 120°C.

During the ageing, the rock sample 28 is maintained under the last capillary pressure obtained during primary drainage at the last first fluid F1 saturation target Swi. This means that downstream valve 74 and upstream valve 72 are closed, while upstream valve 70 and downstream valve 76 are open to maintain the last capillary pressure value.

Upstream valve 70 is connected to the second fluid F2 reservoir of the pressurized second fluid feeder 80.

Downstream valve 76 is connected to the first fluid F1 reservoir of the pressurized first fluid feeder 78.

During the ageing, periodically, one pore volume of second fluid F2 has to be replaced in the rock sample 28 in order to refresh second fluid F2 in the rock sample 28. For example, this refresh is performed 10 times over 1 month of ageing time. To do so, the upstream and downstream valves 72 and 76 are closed while the upstream and downstream valves 70 and 74 are opened. One pore volume of second fluid F2 is injected from the upstream channel 43C through the rock sample 28 and through the downstream channel 56. After each refresh, downstream valve 74 is closed, downstream valve 76 is opened, and the primary drainage capillary pressure at the last first fluid F1 saturation target Swi is reimposed.

The ageing alters the wettability of the rock sample 28 to mimic reservoir conditions, to make it closer to a real reservoir wettability implemented over millions of years.

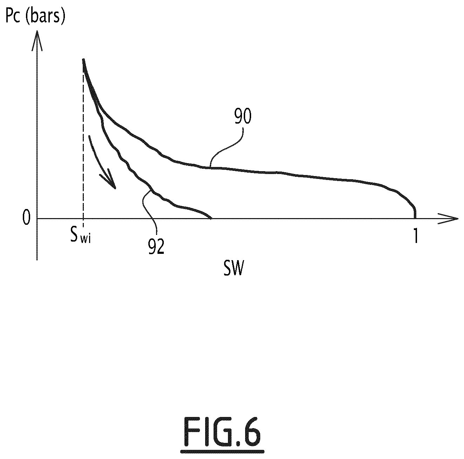

After ageing, the spontaneous imbibition capillary pressure curve 92 shown in figure 6 is determined by carrying out a first fluid F1 imbibition through the rock sample 28 from the downstream face 32 to the upstream face 30.

The second fluid F2 pressure in the rock sample F2 is still larger than the first fluid F1 pressure fed to the rock sample 28.

The first upstream valve 70 is opened, while the second upstream valve 72 is closed. On the contrary, the first downstream valve 74 is closed, while the second downstream valve 76 is opened and connected to a reservoir of first fluid F1 at constant pressure, for example within the first fluid feeder 78.

The pressure in the first upstream channel 45A is then decreased step by step down to a zero capillary pressure where the second fluid F2 pressure in the first upstream channel 45A is equal to the first fluid F1 pressure in the second downstream channel 68.

At each step of pressure decrease, first fluid F1 spontaneously imbibes the rock sample 28 from the downstream face 32 through the downstream porous barrier element 18 which is wet in first fluid F1.

At the end of each step of pressure decrease, when equilibrium is reached, no more first fluid F1 imbibition is observed. The pressure difference between the first upstream measurement point 84A and the second downstream measurement point 86B is representative of the imposed capillary pressure Pc.

At this equilibrium, an image of the rock sample 28 is acquired via the inner structure fluid imaging device 14 to determine an average saturation Sw and/or advantageously a saturation profile along the rock sample 28 and/or 3D distribution of the different fluids in the rock sample 28 pore network.

Thus, the spontaneous imbibition capillary pressure curve 92 depicting capillary pressure Pc as a function of first fluid saturation Sw curve is determined with increasing saturations in first fluid F1 corresponding to decreasing capillary pressures Pc shown by the arrow on Figure 6.

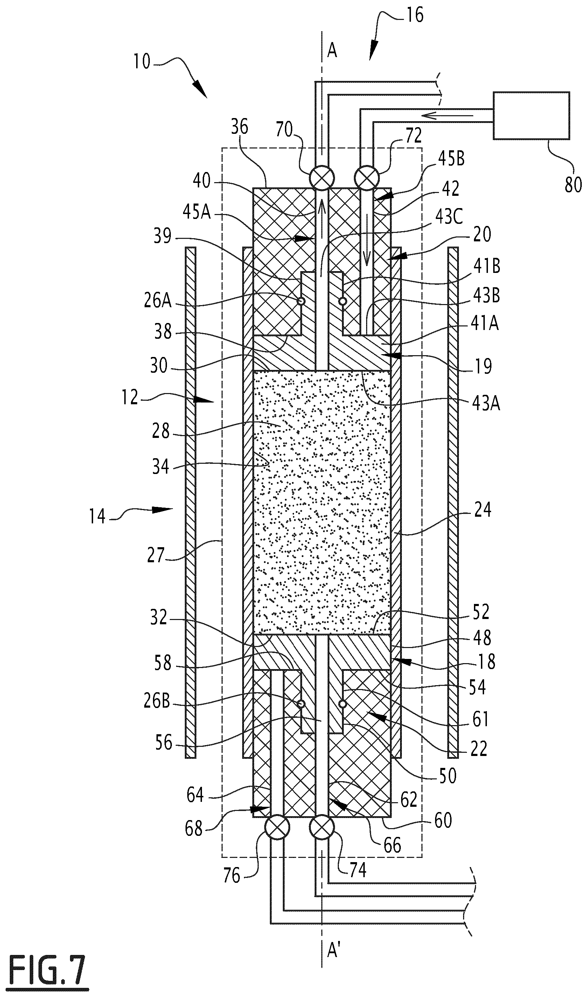

As shown in figure 7, before determining the negative capillary pressure Pc part of the first fluid F1 imbibition curve, second fluid F2 is flushed from the second upstream channel 45B through the upstream porous barrier element 19 to the first upstream channel 45A, without disconnecting or moving the rock properties measurement cell 12 from the fluid distribution and collection device 16 of the rock properties measurement system 10.

The second upstream valve 72 is opened, while the first upstream valve 70 is maintained open. The second downstream valve 76 is closed, while the first downstream valve 74 is maintained closed.

The flushing of second fluid F2 through the upstream porous barrier element 19 traps or removes first fluid F1 from the upstream porous barrier element 19 and installs a second fluid F2 continuity from the downstream face 43A in contact with the upstream face 30 of the rock sample 28 to the upstream channel 45B.

Because of the initial state of full saturation with first fluid F1, residual first fluid F1 may remain in the upstream porous barrier element 19 after flushing. This residual first fluid F1 remains disconnected from the rock sample 28 thanks to the low wettability of the upstream porous barrier element 19 with first fluid F1 and its affinity to second fluid F2.

Thus, after flushing, second fluid F2 remains connected in the upstream porous barrier element 19 and second fluid F2 continuity is then established between the rock sample 28, the upstream porous barrier element 19 and the second upstream channel 45B, when the first upstream channel 45A is closed to the passage of fluid.

Flushing the upstream porous barrier element 19 from the second upstream valve 72 to the first upstream valve 70 avoids over-pressuring the rock sample 28. This prevents unwanted capillary pressure building at the upstream face 30 of the rock sample 28 that could impact fluids distributions within the rock sample 28.

After flushing, the first upstream valve 70 and second upstream valve 72 are closed. Downstream valves 76 and 74 are opened. A second flushing of first fluid F1 through the downstream porous barrier 18 from channel 64 to channel 56 is performed. The main goal of this second flushing is to initialize full 100% saturation in first fluid F1 of the channel 56 down to the channel 86A.

After this second flushing, the second downstream valve 76 is closed. The second upstream valve 72 is opened. The first downstream valve 74 is maintained opened.

The pressurized first fluid feeder 78 is connected to the first downstream channel 66 via the first downstream valve 74. The second upstream channel 45B is connected to a constant pressure reservoir of second fluid F2, e.g. in the pressurized second fluid feeder 80.

First fluid F1 is injected in the rock sample 28 from the pressurized first fluid feeder 78 at various constant pressure steps.

Thanks to the flushing of the upstream porous barrier element 19 with second fluid F2, there is a continuity of second fluid F2 between the rock sample 28 and the upstream porous barrier element 19 to the second upstream channel 45B.

Thus, the first fluid F1 pushes the second fluid F2 in the rock sample 28 and second fluid F2 only exits the rock sample 28 through the upstream porous barrier element 19 to the second upstream channel 45B and the constant pressure reservoir of the second fluid feeder 80, as shown in figure 8.

The pressure difference between the second upstream measurement point 84B and the first downstream measurement point 86A is representative of an imposed capillary pressure Pc which depends on the pressure provided by the pressurized first fluid feeder 78.

At each step of capillary pressure Pc, when no more second fluid F2 is produced in the second upstream channel 45B, an equilibrium capillary pressure Pc is reached in the rock sample 28. At this equilibrium, an image of the sample is acquired via the inner structure fluid imaging device 14 to determine an average saturation Sw in first fluid F1 and/or advantageously a saturation profile in first fluid F1 along the rock sample 28 and/or a 3D saturation mapping in the rock sample 28 pore network.

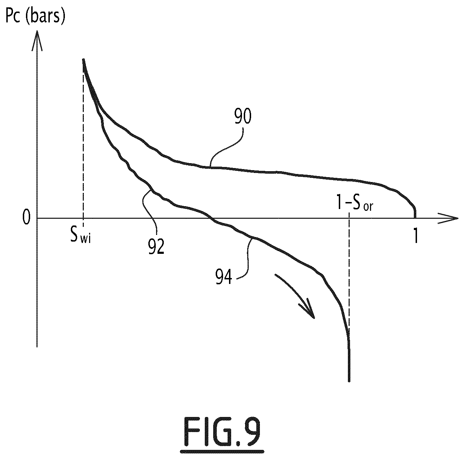

Thus, a negative imbibition segment 94 of the capillary pressure Pc versus first fluid F1 saturation Sw curve is determined with increasing saturations in first fluid F1 corresponding to decreasing capillary pressures Pc shown by the arrow on Figure 9.

After reaching a final first fluid saturation 1-Sor, the second upstream valve 72 is closed and the first upstream valve 70 is opened. First fluid F1 continues to be introduced through the first downstream valve 74 via the downstream bypass 56 and circulates towards the first upstream valve 70 via the upstream bypass 43C.

Multiple flowrates of first fluid F1 introduced in the rock sample 28 are adjusted to be constant and the pressure differences between the first upstream measurement point 84A and the first downstream measurement point 86A is measured to obtain permeability of the rock sample 28 via Darcy's law.

The method according to the invention carried out in the same rock properties measurement cell 12 equipped with a downstream porous barrier element 18 and an upstream porous barrier element 19 thus provides a curve 90 of primary drainage capillary pressure versus saturation in first fluid F1, and curves 92, 94 of respective positive and negative imbibition capillary pressure curve versus saturation in first fluid F1.

The curves 90, 92, 94 are determined from homogeneous saturation profiles taking advantage of the robustness of the downstream porous barrier element 18 and upstream porous barrier element 19 in both primary drainage and imbibition capillary pressure curves determination. If needed, many more cycles of capillary pressure curves could be measured afterwards.

The method uses the same rock properties measurement cell 12 to determine all the curves 90, 92, and 94, without having to remove the rock sample 28 from the rock properties measurement cell 12, and maintaining the pressure applied to the rock sample 28 after each phase of the method.

The use of a rock properties measurement cell 12 having both a downstream porous barrier element 18 and an upstream porous barrier element 19 with different wettabilities to first fluid F1 and second fluid F2 allows building a continuity of first fluid F1 in the downstream porous barrier element 18 and downstream channel 64 and a continuity of second fluid F2 in the upstream porous barrier element 19 and upstream channel 42, via a flushing of second fluid F2 in the upstream porous barrier element 19, while the rock properties measurement cell 12 had been initially fully saturated with first fluid F1.

Experimental conditions in the rock properties measurement cell 12, in particular capillary pressure and temperature remain controlled during the method implementation. The rock properties measurement cell 12 also requires less manual intervention, since the rock sample 28 stays in the cell 12 during the whole implementation. A full capillary pressure curve cycle of the same rock sample 28 is easily obtained, with a given fluid system F1, F2, e.g. oil, brine system (e.g. Oil-Wet, Water-Wet, Mixed-Wet) at experimental conditions.

The rock properties measurement cell 12 allows a measurement of flow properties at end point saturations, to determine permeability or effective permeability. It can handle homogeneous or heterogeneous rocks.

The method according to the invention also provides richer wettability information for simulation, as it directly highlights 3D wettability mapping into the different porosity zones of the rock sample 28, thanks to the 3D in-situ imaging via the inner structure fluid imaging device 14.

## Claims

1. A rock properties measurement cell (12), configured to receive a rock sample (28) to carry out measurements in presence of at least a fluid (F1, F2) contained in said rock sample (28), the measurement cell (12) comprising:
- a downstream porous barrier element (18), permeable to a first fluid (F1) and impervious to a second fluid (F2), the downstream porous barrier element (18) defining an upstream face (52) configured to cooperate with a downstream face (32) of the rock sample (28), a downstream face (54) opposed to the upstream face (52), and a downstream bypass (56) connecting the upstream face (52) and the downstream face (54) of the downstream porous barrier element (18);
- a downstream diffuser (22), comprising:
+ a first downstream opening (62), delimiting, together with the bypass (56), a first downstream channel (66) configured to retrieve first fluid (F1) and/or second fluid (F2) from the rock sample (28) or to inject first fluid (F1) and/or second fluid (F2) in the rock sample (28),
+ a second downstream opening (64) forming a second downstream channel (68), opening facing the downstream face (54) of the downstream porous barrier element (18) and being configured to retrieve only first fluid (F1) from the rock sample (28) or to inject only first fluid (F1) in the rock sample (28),
- an upstream diffuser (20);
- a sleeve (24) ;
**characterized by** an upstream porous barrier element (19), impervious to the first fluid (F1) and permeable to the second fluid (F2), the upstream porous barrier element (19) defining an downstream face (43A) configured to cooperate with a upstream face (30) of the rock sample (28), an upstream face (43B) opposed to the downstream face (43A), and a upstream bypass (43C) connecting the upstream face (43B) and the downstream face (43A) of the upstream porous barrier element (19);
the upstream diffuser (20) comprising:
+ a first upstream opening (40), delimiting, together with the upstream bypass (43C), a first upstream channel (45A) configured to retrieve first fluid (F1) and/or second fluid (F2) from the rock sample (28) or to inject first fluid (F1) and/or second fluid (F2) in the rock sample (28) via the upstream bypass (43C),
+ a second upstream opening (42) forming a second upstream channel (45B), opening facing the upstream face (30) of the upstream porous barrier (19) element and being configured to retrieve only second fluid (F2) from the rock sample (28) or to inject only second fluid (F2) in the rock sample (28),
the upstream diffuser (20), the upstream porous barrier element (19), the downstream porous barrier element (18), the downstream diffuser (22) and the sleeve (24) surrounding them delimiting a measurement chamber (34) configured to receive the rock sample (28).

2. The rock properties measurement cell (12) according to claim 1, wherein the upstream porous barrier element (19) comprises:
+ a downstream segment (41A) of a first diameter (D1), the downstream segment (41A) defining the downstream face (43A) configured to cooperate with the rock sample (28),
+ an upstream segment (41B) of a second diameter (D2) smaller than the first diameter (D1), the upstream segment (41B) and the downstream segment (41A) defining together the upstream face (43B) opposed to the downstream face (43A), the upstream bypass (43C) connecting the upstream face (43B) and the downstream face (43A) of the upstream porous barrier element (19) through the upstream segment (41B) and the downstream segment (41A).

3. The rock properties measurement cell (12) according to claim 2, wherein the upstream diffuser (20) defines an upstream cavity (39), the upstream segment (41B) of the upstream porous barrier element (19) being received in the upstream cavity (39), the measurement cell (12) comprising an upstream sealing gasket (26A) arranged around the upstream segment (41B) of the upstream porous barrier element (19) between the upstream segment (41B) and the upstream diffuser (20).

4. The rock properties measurement cell (12) according to any one of claims 2 or 3, wherein the downstream porous barrier element (18) comprises:
+ an upstream segment (48) of a first diameter (D1), the upstream segment (48) defining the upstream face (52) configured to cooperate with a downstream face (32) of the rock sample (28),
+ a downstream segment (50) of a second diameter (D2) smaller than the first diameter (D1), the upstream segment (48) and the downstream segment (50) defining together the downstream face (54) opposed to the upstream face (52),
the downstream bypass (56) connecting the upstream face (52) and the downstream face (54) of the downstream porous barrier element (18) through the upstream segment (48) and through the downstream segment (50) of the downstream porous barrier element (18), the upstream segment (41B) of the upstream porous barrier element (19) and the downstream segment (50) of the downstream porous barrier element (18) protruding in opposed longitudinal directions of the rock properties measurement cell (12).

5. The rock properties measurement cell (12) according to any one of the preceding claims, wherein the wettability of the downstream porous barrier element (18) to the first fluid (F1) is greater than the wettability of the upstream porous barrier element (19) to the first fluid (F1), the wettability of the downstream porous barrier element (18) to the second fluid (F2) is smaller than the wettability of the upstream porous barrier element (19) to the second fluid (F2).

6. A rock properties measurement system (10) comprising a rock properties measurement cell (12) according to any one of claims 1 to 5, wherein the system (10) comprises a fluid distribution and collection device (16) connected to the measurement cell (12), configured to selectively feed the first fluid (F1) and the second fluid (F2) to the rock properties measurement cell (12).

7. The rock properties measurement system (10) according to claim 6, comprising:
- a first upstream valve (70), fluidly connected to the first upstream opening (40), and configured to be actuated between an open position freeing fluid passage through the first upstream opening (40) to feed first fluid (F1) and/or second fluid (F2) to the first upstream channel (45A) or retrieve first fluid (F1) and/or second fluid (F2) from the first upstream channel (45A), and a closed position, blocking fluid passage through the first upstream opening (40);
- a second upstream valve (72), fluidly connected to the second upstream opening (42), and configured to be actuated between an open position freeing fluid passage through the second upstream opening (42) to feed second fluid (F2) to the second upstream channel (45B) or retrieve second fluid (F2) from the second upstream channel (45B), and a closed position, blocking fluid passage through the second upstream opening (42);
- a first downstream valve (74), fluidly connected to the first downstream opening (62), and configured to be actuated between an open position freeing fluid passage through the first downstream opening (62) to feed first fluid (F1) and/or second fluid (F2) to the first downstream channel (66) or retrieve first fluid (F1) and/or second fluid (F2) from the first downstream channel (66), and a closed position, blocking fluid passage through the first downstream opening (62);
- a second downstream valve (76), fluidly connected to the second downstream opening (64), and configured to be actuated between an open position freeing fluid passage through the second downstream opening (64) to feed first fluid (F1) to the second downstream channel (68) or retrieve first fluid (F1) from the second downstream channel (68), and a closed position, blocking fluid passage through the second upstream opening (64).

8. The rock properties measurement system (10) according to claims 6 or 7, comprising a pressure difference measuring system (82) configured to be selectively connected between (i) at least an upstream pressure measurement point (84A, 84B) representative of the pressure in one of the first upstream channel (45A) and of the second upstream channel (45B), (ii) at least a downstream pressure measurement point (86A, 86B) representative of the pressure in one of the first downstream channel (66) and of the second downstream channel (68).

9. The rock properties measurement system (10) according to any one of claims 6 to 8, comprising an inner structure fluid imaging device (14) arranged at an outside of the rock properties measurement cell (12), wherein the inner structure imaging device (14) is advantageously a microtomography imaging device or nuclear magnetic resonance imaging device.

10. The rock properties measurement system (10) according to any one of claims 6 to 9, comprising a first fluid or second fluid pressurized feeder (78), configured to be selectively connected to the first upstream channel (45A) and/or to the second upstream channel (45B), and comprising a first fluid or second fluid pressurized feeder (80), configured to be selectively connected to the first downstream channel (66) and/or to the second downstream channel (68).

11. A rock properties measurement method, comprising:
- providing a rock sample containing a saturation (Sw) of a second fluid (F2) in a rock properties measurement cell (12) of a rock properties measurement system (10) according to any one of claims 6 to 10, the downstream face (43A) of the upstream porous barrier element (19) being applied on an upstream face (30) of the rock sample (28), the upstream face (52) of the downstream porous barrier element (18) being applied on an downstream face (32) of the rock sample (28);
- injecting first fluid (F1) in the rock sample (28) through the first downstream channel (66), and recovering second fluid (F2) through the second upstream channel (45B), the first upstream channel (45A) being blocked to fluid passage, the second upstream channel (45B) being advantageously connected to a second fluid constant pressure reservoir ;
- during or/and after the injecting of first fluid (F1) in the rock sample (28) through the first downstream channel (66), measuring at least a rock sample property, in particular at least one of a capillary pressure in the rock sample (28) during and/or after injection of the first fluid (F1) and a fluid saturation in the rock sample (28) during and/or after injection of the first fluid (F1).

12. The rock properties measurement method according to claim 11, comprising, before injecting first fluid (F1) in the rock sample (28) through the first downstream channel (66), flushing second fluid (F2) from the second upstream channel (45B) through the upstream porous barrier element (19) to the first upstream channel (45A), the injecting of first fluid (F1) in the rock sample (28) through the first downstream channel (66) being carried out after the flushing of second fluid (F2), without disconnecting or moving the rock properties measurement cell (12) from the fluid distribution and collection device (16) of the rock properties measurement system (10).

13. The rock properties measurement method according to any one of claims 11 or 12, wherein providing a rock sample (28) containing a saturation (Sw) of a second fluid (F2) in a rock properties measurement cell (12) comprises:
- draining second fluid (F2) in the rock sample (28) containing first fluid (F1), through the first upstream channel (45A), and recovering first fluid (F1) through the second downstream channel (68), the first downstream channel (66) being blocked to fluid passage, the second downstream channel (68) being advantageously connected to a first fluid constant pressure reservoir, in order to reach a target first fluid (F1) saturation and/or capillary pressure, and measuring at least a rock sample property, in particular at least one of a capillary pressure in the rock sample (28) during and/or after draining of the second fluid (F2) and a fluid saturation in the rock sample (28) during and/or after draining of the second fluid (F2) and/or :
- imbibing first fluid (F1) in the rock sample (28) containing second fluid (F2), through the second downstream channel (68), the second downstream channel (68) being advantageously connected to a first fluid constant pressure reservoir and recovering first fluid (F1) and/or second fluid (F2) through the first upstream channel (45A), measuring at least a rock sample property, in particular at least one of a capillary pressure in the rock sample (28) during and/or after imbibition of the first fluid (F1) and a fluid saturation in the rock sample (28) during and/or after imbibition of the first fluid (F1),
the injecting of first fluid (F1) in the rock sample (28) through the first downstream channel (66) being carried out after the imbibing of first fluid (F1), without disconnecting or moving the rock properties measurement cell (12) from the fluid distribution and collection device (16) of the rock properties measurement system (10).

14. The rock properties measurement method according to claim 13, comprising, after draining second fluid (F2) in the rock sample (28), placing the rock properties measurement cell (12) in an oven to heat the rock properties measurement cell (12) while keeping connections with the fluid distribution and collection device (16) and maintaining the target first fluid (F1) saturation and/or capillary pressure.

15. The rock properties measurement method according to any one of claims 11 to 14, wherein the first fluid (F1) is water, the second fluid (F2) being oil.
